(19) Europäisches Patentamt
European Patent Office
Office européen des brevets

(11) Veröffentlichungsnummer: **0 024 038**
**B1**

(12) # EUROPÄISCHE PATENTSCHRIFT

(45) Veröffentlichungstag der Patentschrift: **13.04.83**

(51) Int. Cl.³: **C 07 D 487/04,** A 61 K 31/55 // (C07D487/04, 243/00, 231/00)

(21) Anmeldenummer: **80104654.1**

(22) Anmeldetag: **07.08.80**

(54) **4-Phenyl-5,6,7,8-tetrahydropyrazolo(3,4-b)-(1,5)-diazepin-1H,4H-5,7-dione, Verfahren zu ihrer Darstellung und diese enthaltende Arzneimittel.**

(30) Priorität: **14.08.79 DE 2932835**

(43) Veröffentlichungstag der Anmeldung: **18.02.81 Patentblatt 81/7**

(45) Bekanntmachung des Hinweises auf die Patenterteilung: **13.04.83 Patentblatt 83/15**

(84) Benannte Vertragsstaaten: **AT BE CH DE FR GB IT LI NL SE**

(56) Entgegenhaltungen: **DE-A-2 360 852**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt/Main 80 (DE)**

(72) Erfinder: **Rackur, Gerhard, Dr., Gundelhardtstrasse 2, D-6233 Kelkheim (Taunus) (DE)**
Erfinder: **Hoffmann, Irmgard, Dr., Oranienstrasse 1, D-6232 Bad Soden am Taunus (DE)**

**EP 0 024 038 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# 4-Phenyl-5,6,7,8-tetrahydropyrazolo(3,4-b)(1,5)diazepin-1H,4H-5,7-dione, Verfahren zu ihrer Darstellung und diese enthaltende Arzneimittel

Die Erfindung betrifft neue 4-Phenyl-5,6,7,8-tetrahydropyrazolo(3,4-b)(1,5)diazepin-1H,4H-5,7-dione der allgemeinen Formel I

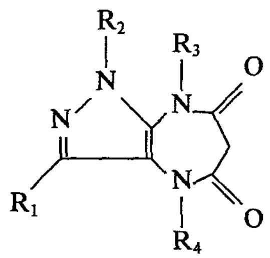

(I)

worin

- $R_1$ und $R_2$ gleich oder verschieden sind und Wasserstoffatome, Alkylgruppen mit 1–6 C-Atomen darstellen, wobei einer der Reste $R_1$ und $R_2$ auch eine Benzyl-, Trifluormethyl- oder Phenylgruppe sein kann,
- $R_3$ ein Wasserstoffatom, eine Alkylgruppe mit 1–6 C-Atomen, die gegebenenfalls durch eine Phenylgruppe, eine Alkoxygruppe mit 1–6 C-Atomen, eine Trifluormehtyl- oder eine Dialkylaminogruppe mit 2–12 C-Atomen oder eine Cycloalkylgruppe mit 3–6 C-Atomen substituiert ist, eine Alkenyl- oder Alkinylgruppe mit 2–6 C-Atomen, eine Cycloalkylgruppe mit 3–6 C-Atomen, eine Carbalkoxygruppe mit 2–6 C-Atomen bedeutet und
- $R_4$ eine Phenylgruppe, eine ein- oder zweifach durch Methyl, Cl, Br, F, Nitro, Cyan und/oder Trifluormethyl substituierte Phenylgruppe

bedeutet.

Insbesondere beinhaltet die Erfindung Verbindungen, worin $R_1$ und $R_2$ gleich oder verschieden sind und Wasserstoff, Methyl, Ethyl, iso-Propyl oder n-Butyl bedeuten, $R_2$ gegebenenfalls vorteilhaft ein Phenyl- oder Benzylrest sein kann.

Für den Rest $R_3$ kommt insbesondere in Betracht ein Wasserstoffatom, eine Methyl-, Ethyl-, Benzyl-, Methoxy-, Dimethoxy- oder Trimethoxybenzyl-, Propenyl-, Propinyl-, Cyclopropylmethylgruppe, eine Methoxymethylen- sowie eine Ethoxymethylengruppierung.

Für $R_4$ kommen insbesondere Phenyl, 2-Cl-Phenyl, 3-Cl-Phenyl, 4-Cl-Phenyl, 2,4-Dichlorphenyl sowie die entsprechenden Fluorderivate in Betracht.

Besonders günstige Eigenschaften besitzen solche Verbindungen der allgemeinen Formel I, bei denen $R_2$ Methyl, Ethyl oder Phenyl bedeutet, $R_1$ Methyl ist, $R_3$ Wasserstoff, Methyl, Ethyl, Cyclopropylmethyl oder Propinyl ist und $R_4$ ein Phenyl- oder 2- oder 3-Chlor- bzw. Fluorphenylrest ist.

Verbindungen gemäß der Erfindung sind beispielsweise:

1-Methyl-4-phenyl-5,6,7,8-tetrahydropyrazolo(3,4-b)(1,5)diazepin-1H,4H-5,7-dion,
1,8-Dimethyl-4-phenyl-5,6,7,8-tetrahydropyrazolo(3,4-b)(1,5)diazepin-1H,4H-5,7-dion,
8-Ethyl-1-methyl-4-phenyl-5,6,7,8-tetrahydropyrazolo(3,4-b)(1,5)diazepin-1H,4H-5,7-dion,
8-Allyl-1-methyl-4-phenyl-5,6,7,8-tetrahydropyrazolo(3,4-b)(1,5)diazepin-1H,4H-5,7-dion,
1-Methyl-8-(2-propinyl)-4-phenyl-5,6,7,8-tetrahydro(3,4-b)(1,5)diazepin-1H,4H-5,7-dion,
8-Cyclopropylmethyl-1-methyl-4-phenyl-5,6,7,8-tetrahydropyrazolo(3,4-b)-(1,5)diazepin-1H,4H-5,7-dion,
1-Methyl-8-(2-dimethylaminoethyl)-4-phenyl-5,6,7,8-tetrahydropyrazolo(3,4-b)-(1,5)diazepin-1H,4H-5,7-dion,
8-(2-Diethylaminoethyl)-1-methyl-4-phenyl-5,6,7,8-tetrahydropyrazolo(3,4-b)-(1,5)diazepin-1H,4H-5,7-dion,
8-(2,2,2-trifluorethyl)-1-methyl-4-phenyl-5,6,7,8-tetrahydropyrazolo(3,4-b)-(1,5)diazepin-1H,4H-5,7-dion,
1,3-Dimethyl-8-ethyl-4-phenyl-5,6,7,8-tetrahydropyrazolo(3,4-b)-(1,5)diazepin-1H,4H-5,7-dion,
8-Allyl-1,3-dimethyl-4-phenyl-5,6,7,8-tetrahydropyrazolo(3,4-b)(1,5)diazepin-1H,4H-5,7-dion,
1,3-Dimethyl-8-(2-propinyl)-4-phenyl-5,6,7,8-tetrahydropyrazolo(3,4-b)-(1,5)diazepin-1H,4H-5,7-dion,

8-Allyl-1,3-dimethyl-4-(3,4-dichlorphenyl)-5,6,7,8-tetrahydropyrazolo(3,4-b)-(1,5)diazepin-1H,4H-5,7-dion,
1,3-Dimethyl-8-(2-propinyl)-4-(4-chlorphenyl)-5,6,7,8-tetrahydropyrazolo(3,4-b)-(1,5)diazepin-1H,4H-5,7-dion,
1,3-Dimethyl-8-cyclopropylmethyl-4-(p-tolyl)-5,6,7,8-tetrahydropyrazolo(3,4-b)-(1,5)diazepin-1H,4H-5,7-dion,
1,3-Dimethyl-8-(2-dimethylaminoethyl)-4-(2-chlorphenyl)-5,6,7,8-tetrahydro-pyrazolo(3,4-b)(1,5)diazepin-1H,4H-5,7-dion,
1,3-Dimethyl-8-(2-diethylaminoethyl)-4-(2,4-dichlorphenyl)-5,6,7,8-tetrahydro-pyrazolo(3,4-b)(1,5)diazepin-1H,4H-5,7-dion,
1,3-Dimethyl-8-(2,2,2-trifluorethyl)-4-(2-cyanophenyl)-5,6,7,8-tetrahydro-pyrazolo(3,4-b)(1,5)diazepin-1H,4H-5,7-dion,
1-Ethyl-3,8-dimethyl-4-(2-chlorphenyl)-5,6,7,8-tetrahydropyrazolo(3,4-b)-(1,5)diazepin-1H,4H-5,7-dion,
8-Allyl-1-ethyl-3-methyl-4-(2,4-dichlorphenyl)-5,6,7,8-tetrahydropyrazolo(3,4-b)-(1,5)diazepin-1H,4H-5,7-dion,
8-Cyclopropylmethyl-1-ethyl-3-methyl-4-(2-trifluormethylphenyl)-5,6,7,8-tetra-hydropyrazolo(3,4-b)(1,5)diazepin-1H,4H-5,7-dion,
1-Ethyl-3-methyl-8-(2-dimethylaminoethyl)-4-(o-tolyl)-5,6,7,8-tetrahydro-pyrazolo(3,4-b)(1,5)diazepin-1H,4H-5,7-dion,
1-Phenyl-3,8-dimethyl-4-(m-tolyl)-5,6,7,8-tetrahydropyrazolo(3,4-b)-(1,5)diazepin-1H,4H-5,7-dion,
8-Allyl-3-methyl-1-phenyl-4-(3,4-dichlorphenyl)-5,6,7,8-tetrahydropyrazolo(3,4-b)-(1,5)diazepin-1H,4H-5,7-dion,
3-Methyl-1-phenyl-8-(2-propinyl)-4-(3-chlorphenyl)-5,6,7,8-tetrahydropyrazolo(3,4-b)-(1,5)diazepin-1H,4H-5,7-dion,
8-Cyclopropylmethyl-3-methyl-1-phenyl-4-(2-cyanophenyl-5,6,7,8-tetrahydro-pyrazolo(3,4-b)(1,5)diazepin-1H,4H-5,7-dion,
8-(2-Dimethylaminoethyl)-3-methyl-1-phenyl-4-(2-chlorphenyl)-5,6,7,8-tetra-hydropyrazolo(3,4-b)(1,5)diazepin-1H,4H-5,7-dion,
8-(2,2,2-Trifluorethyl)-3-methyl-1-phenyl-4-(o-tolyl)-5,6,7,8-tetrahydro-pyrazolo(3,4-b)(1,5)diazepin-1H,4H-5,7-dion,
1-Benzyl-3,8-dimethyl-4-(2-chlorphenyl)-5,6,7,8-tetrahydropyrazolo(3,4-b)-(1,5)diazepin-1H,4H-5,7-dion,
8-Allyl-1-benzyl-3-methyl-4-(3,4-dichlorphenyl)-5,6,7,8-tetrahydropyrazolo(3,4-b)-(1,5)diazepin-1H,4H-5,7-dion,
1-Benzyl-3-methyl-8-(2-propinyl)-4-(o-tolyl)-5,6,7,8-tetrahydropyrazolo(3,4-b)-(1,5)diazepin-1H,4H-5,7-dion,
1-Benzyl-8-cyclopropylmethyl-3-methyl-4-(4-chlorphenyl)-5,6,7,8-tetrahydro-pyrazolo(3,4-b)(1,5)diazepin-1H,4H-5,7-dion,
1-Benzyl-3-methyl-8-(2-dimethylaminoethyl)-4-(3-chlorphenyl)-5,6,7,8-tetrahydro-pyrazolo(3,4-b)(1,5)diazepin-1H,4H-5,7-dion,
1-Benzyl-3-methyl-8-(2-diethylaminoethyl)-4-(2-trifluormethylphenyl)-5,6,7,8-tetra-hydropyrazolo(3,4-b)(1,5)diazepin-1H,4H-5,7-dion,
1-Benzyl-3-methyl-8-(2,2,2-trifluorethyl)-4-(4-chlorphenyl)-5,6,7,8-tetrahydro-pyrazolo(3,4-b)(1,5)diazepin-1H,4H-5,7-dion.

Die erfindungsgemäßen Verbindungen besitzen hervorragende pharmakologische Eigenschaften, insbesondere wirken sie anxiolytisch und antikonvulsiv, zum Teil auch antiphlogistisch.

Gegenstand der Erfindung sind weiterhin ein Verfahren zu ihrer Herstellung sowie pharmazeutische Zubereitungen dieser Verbindungen und ihre Verwendung als Arzneimittel.

Die erfindungsgemäßen Verbindungen werden hergestellt, indem man eine Verbindung der allgemeinen Formel II

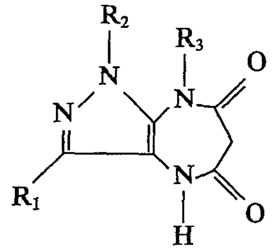

(II)

(1,5)diazepin-1H,4H-5,7-dion,
8-Allyl-1,3-dimethyl-4-(3,4-dichlorphenyl)-5,6,7,8-tetrahydropyrazolo(3,4-b)-(1,5)diazepin-1H,4H-5,7-dion,
1,3-Dimethyl-8-(2-propinyl)-4-(4-chlorphenyl)-5,6,7,8-tetrahydropyrazolo(3,4-b)-(1,5)diazepin-1H,4H-5,7-dion,
1,3-Dimethyl-8-cyclopropylmethyl-4-(p-tolyl)-5,6,7,8-tetrahydropyrazolo(3,4-b)-(1,5)diazepin-1H,4H-5,7-dion,
1,3-Dimethyl-8-(2-dimethylaminoethyl)-4-(2-chlorphenyl)-5,6,7,8-tetrahydro-pyrazolo(3,4-b)(1,5)diazepin-1H,4H-5,7-dion,
1,3-Dimethyl-8-(2-diethylaminoethyl)-4-(2,4-dichlorphenyl)-5,6,7,8-tetrahydro-pyrazolo(3,4-b)(1,5)diazepin-1H,4H-5,7-dion,
1,3-Dimethyl-8-(2,2,2-trifluorethyl)-4-(2-cyanophenyl)-5,6,7,8-tetrahydro-pyrazolo(3,4-b)(1,5)diazepin-1H,4H-5,7-dion,
1-Ethyl-3,8-dimethyl-4-(2-chlorphenyl)-5,6,7,8-tetrahydropyrazolo(3,4-b)-(1,5)diazepin-1H,4H-5,7-dion,
8-Allyl-1-ethyl-3-methyl-4-(2,4-dichlorphenyl)-5,6,7,8-tetrahydropyrazolo(3,4-b)-(1,5)diazepin-1H,4H-5,7-dion,
8-Cyclopropylmethyl-1-ethyl-3-methyl-4-(2-trifluormethylphenyl)-5,6,7,8-tetra-hydropyrazolo(3,4-b)(1,5)diazepin-1H,4H-5,7-dion,
1-Ethyl-3-methyl-8-(2-dimethylaminoethyl)-4-(o-tolyl)-5,6,7,8-tetrahydro-pyrazolo(3,4-b)(1,5)diazepin-1H,4H-5,7-dion,
1-Phenyl-3,8-dimethyl-4-(m-tolyl)-5,6,7,8-tetrahydropyrazolo(3,4-b)-(1,5)diazepin-1H,4H-5,7-dion,
8-Allyl-3-methyl-1-phenyl-4-(3,4-dichlorphenyl)-5,6,7,8-tetrahydropyrazolo(3,4-b)-(1,5)diazepin-1H,4H-5,7-dion,
3-Methyl-1-phenyl-8-(2-propinyl)-4-(3-chlorphenyl)-5,6,7,8-tetrahydropyrazolo(3,4-b)-(1,5)diazepin-1H,4H-5,7-dion,
8-Cyclopropylmethyl-3-methyl-1-phenyl-4-(2-cyanophenyl-5,6,7,8-tetrahydro-pyrazolo(3,4-b)(1,5)diazepin-1H,4H-5,7-dion,
8-(2-Dimethylaminoethyl)-3-methyl-1-phenyl-4-(2-chlorphenyl)-5,6,7,8-tetra-hydropyrazolo(3,4-b)(1,5)diazepin-1H,4H-5,7-dion,
8-(2,2,2-Trifluorethyl)-3-methyl-1-phenyl-4-(o-tolyl)-5,6,7,8-tetrahydro-pyrazolo(3,4-b)(1,5)diazepin-1H,4H-5,7-dion,
1-Benzyl-3,8-dimethyl-4-(2-chlorphenyl)-5,6,7,8-tetrahydropyrazolo(3,4-b)-(1,5)diazepin-1H,4H-5,7-dion,
8-Allyl-1-benzyl-3-methyl-4-(3,4-dichlorphenyl)-5,6,7,8-tetrahydropyrazolo(3,4-b)-(1,5)diazepin-1H,4H-5,7-dion,
1-Benzyl-3-methyl-8-(2-propinyl)-4-(o-tolyl)-5,6,7,8-tetrahydropyrazolo(3,4-b)-(1,5)diazepin-1H,4H-5,7-dion,
1-Benzyl-8-cyclopropylmethyl-3-methyl-4-(4-chlorphenyl)-5,6,7,8-tetrahydro-pyrazolo(3,4-b)(1,5)diazepin-1H,4H-5,7-dion,
1-Benzyl-3-methyl-8-(2-dimethylaminoethyl)-4-(3-chlorphenyl)-5,6,7,8-tetrahydro-pyrazolo(3,4-b)(1,5)diazepin-1H,4H-5,7-dion,
1-Benzyl-3-methyl-8-(2-diethylaminoethyl)-4-(2-trifluormethylphenyl)-5,6,7,8-tetra-hydropyrazolo(3,4-b)(1,5)diazepin-1H,4H-5,7-dion,
1-Benzyl-3-methyl-8-(2,2,2-trifluorethyl)-4-(4-chlorphenyl)-5,6,7,8-tetrahydro-pyrazolo(3,4-b)(1,5)diazepin-1H,4H-5,7-dion.

Die erfindungsgemäßen Verbindungen besitzen hervorragende pharmakologische Eigenschaften, insbesondere wirken sie anxiolytisch und antikonvulsiv, zum Teil auch antiphlogistisch.

Gegenstand der Erfindung sind weiterhin ein Verfahren zu ihrer Herstellung sowie pharmazeutische Zubereitungen dieser Verbindungen und ihre Verwendung als Arzneimittel.

Die erfindungsgemäßen Verbindungen werden hergestellt, indem man eine Verbindung der allgemeinen Formel II

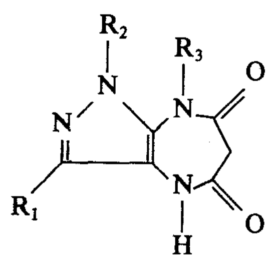

(II)

am Stickstoff in der 4-Position nach einem Verfahren phenyliert, das zuerst von I. Goldberg [Ber. 40, 4541 (1907)] zur Darstellung von N-Acetyldiarylaminen beschrieben wurde. Danach wird die Verbindung II mit einer Verbindung der allgemeinen Formel III

$R_4X$ (III)

umgesetzt, wobei $R_4$ die oben angegebenen Bedeutungen hat und X ein Halogenatom, vorzugsweise Chlor oder Brom, ist.

Die für dieses Verfahren erforderlichen Ausgangssubstanzen der allgemeinen Formel II sind Gegenstand der europäischen Teilanmeldung 81 110 770.5.

Die Umsetzung erfolgt in Gegenwart von Kupferpulver, Kupfer(I)- oder Kupfer(II)-salzen oder Mischungen derselben, entweder unter Verwendung des Halogenids der allgemeinen Formel III im Überschuß oder in polaren aprotischen Lösungsmitteln, wie z. B. Dimethylformamid, Dimethylsulfoxid oder Hexamethylphosphorsäuretriamid. Arbeitet man in Lösungsmitteln, so wird das Halogenid lediglich in etwas mehr als der äquimolaren Menge zugesetzt. Die Reaktionstemperatur ist abhängig von den jeweils eingesetzten Ausgangsstoffen und liegt im allgemeinen zwischen 90 und 180° C. Als basischer Katalysator sowie als Base zur Bindung des entstehenden Halogenwasserstoffes ist der Zusatz einer geeigneten organischen oder anorganischen Base, beispielsweise eines Alkalicarbonats, -bicarbonats oder -alkoholats, vorzugsweise eines Alkaliacetats in molaren Mengen oder im Überschuß notwendig.

Verbindungen der allgemeinen Formel I, worin $R_1$, $R_2$ und $R_4$ die oben angegebenen Bedeutungen haben und $R_3$ ein Wasserstoffatom ist, lassen sich beispielsweise auf folgendem Weg darstellen: Man synthetisiert, wie beschrieben, eine Verbindung der allgemeinen Formel I, wobei $R_1$, $R_2$ und $R_4$ die oben angegebenen Bedeutungen haben und $R_3$ ein Rest ist, der sich nach beendeter Synthese abspalten läßt. Dazu kommen beispielsweise in Frage der Benzylrest, der hydrogenolytisch abgespalten werden kann, oder der Methoxy-, Dimethoxy- oder Trimethoxybenzylrest, der unter sauren Bedingungen abspaltbar ist. Die katalytische Hydrierung kann beispielsweise mit Pd, Pt oder Raney-Nickel in Alkoholen, Dioxan, THF oder Essigester bei 60—150° C und 1—150 Atmosphären Wasserstoff-Druck erfolgen. Die saure Abspaltung kann in Gegenwart von Mineralsäure wie HCl, $H_2SO_4$ in organischen Lösungsmitteln, z. B. Alkoholen, oder starken organischen Säuren wie Trifluoressigsäure oder Toluolsulfonsäure in organischen Lösungsmitteln wie Alkoholen oder chlorierten Kohlenwasserstoffen durchgeführt werden.

Die erfindungsgemäßen Verbindungen sind zur Herstellung von Arzneimitteln geeignet. Die Arzneimittel können eine oder mehrere der erfindungsgemäßen Verbindungen oder auch Mischungen derselben mit anderen pharmazeutisch wirksamen Stoffen enthalten. Zur Herstellung der Arzneimittel können die üblichen pharmazeutischen Träger und Hilfsstoffe und an sich bekannte galenische Verfahren verwendet werden. Die Arzneimittel können enteral, parenteral, oral oder perlingual angewendet werden. Beispielsweise kann die Verabreichung in Form von Tabletten, Kapseln, Pillen, Dragees, Zäpfchen, Gelees, Cremes, Puder, Liquida, Stäubepulver oder Aerosolen erfolgen. Als Liquida kommen zum Beispiel ölige oder wäßrige Lösungen oder Suspensionen, Emulsionen und injizierbare wäßrige Lösungen oder Suspensionen in Betracht.

Die erfindungsgemäßen Verbindungen besitzen hervorragende pharmakologische Eigenschaften, insbesondere wirken sie anxiolytisch und antikonvulsiv, zum Teil auch antiphlogistisch. Sie sind deshalb zur Behandlung von Schlaflosigkeit, Erregung und vegetativer Verstimmung geeignet.

Die pharmazeutischen Zubereitungen enthalten im allgemeinen zwischen 1 bis 10% der erfindungsgemäßen aktiven Komponente(n).

Die anxiolytische Wirkung ist von einer sehr guten Verträglichkeit begleitet ($LD_{50}$ i.a. $\geqslant$1200 mg/kg p.o. an der Maus). Die niedrigste, bereits wirksame Dosis in den oben angegebenen Versuchen ist beispielsweise 5 mg/kg oral, 2,5 mg/kg sublingual, 1 mg/kg intravenös. Als allgemeiner Dosisbereich für Wirkung (Tierversuch wie oben) kommt beispielsweise in Frage: 5 bis 50 mg/kg oral, 2,5 bis 25 mg/kg sublingual, 1 bis 10 mg/kg intravenös.

Beispielsweise können 3mal täglich 1 bis 3 Tabletten mit einem Gehalt von 10 bis 100 mg wirksamer Substanz oder zum Beispiel bei intravenöser Injektion 1- bis 3mal täglich eine Ampulle von 2 bis 4 ml Inhalt mit 0,5 bis 5 mg Substanz empfohlen werden. Die maximale tägliche Dosis soll jedoch nicht über 200 mg liegen.

Darüber hinaus haben diese Substanzen eine nootrope Wirkung, die in verschiedenen Testmethoden nachgewiesen werden konnte. Im One-Trial-Dark-Avoidance Test ist die Substanz von Beispiel 1 20—40mal und die Substanz von Beispiel 31 bis zu 80mal wirksamer als die Vergleichssubstanz Piracetam. Die niedrigste bereits wirksame Dosis in diesem Test ist 12,5 mg/kg p.o. bei der Substanz von Beispiel 1 und 6,3 mg/kg p.o. bei der Substanz von Beispiel 31, während bei der Vergleichssubstanz Piracetam die niedrigste wirksame Dosis bei 500 mg/kg p.o. liegt. Eine weitere deutliche Wirksamkeit konnte beim Gamma-Butyrolacton-Antagonismus, dem Brevatonal-Antagonismus an Ratte und Maus beobachtet werden. Als weiterer Versuch zur Charakterisierung der nootropen Eigenschaft dieser Substanz wurde der Erstickungs-Test an Mäusen herangezogen.

## Beispiel 1

### 1,3,8-Trimethyl-4-phenyl-5,6,7,8-tetrahydropyrazolo(3,4-b)(1,5)diazepin-1H,4H-5,7-dion

Ein Gemisch von 1 g 1,3,8-Trimethyl-5,6,7,8-tetrahydropyrazolo(3,4-b)(1,5)diazepin-1H,4H-5,7-dion, 1 g Kaliumacetat und 1,5 g Kupferpulver wird in 100 ml Brombenzol unter Rühren so lange unter Rückfluß gekocht, bis die Reaktion beendet ist (DC-Kontrolle, 3–4 Stunden). Anschließend läßt man auf Raumtemperatur abkühlen, verdünnt mit $CH_2Cl_2$ (200 ml), filtriert von den anorganischen Bestandteilen ab, wäscht die organische Phase mit Wasser, trocknet und zieht das Lösungsmittel im Vakuum ab. Der Rückstand ergibt nach Umkristallisation aus Diisopropylether das analysenreine Produkt.
Fp.: 188°C.

## Beispiel 2

### 1-Ethyl-3,8-dimethyl-4-phenyl-5,6,7,8-tetrahydropyrazolo(3,4-b)(1,5)diazepin-1H,4H-5,7-dion

Die Umsetzung von 1-Ethyl-3,8-dimethyl-5,6,7,8-tetrahydropyrazolo(3,4-b)(1,5)diazepin-1H,4H-5,7-dion mit Brombenzol, Kupferpulver und Kaliumacetat erfolgt nach der in Beispiel 1 beschriebenen Methode. Umkristallisation aus Diisopropylether ergibt das analysenreine Produkt.
Fp.: 149°C.

## Beispiel 3

### 1-Isopropyl-3,8-dimethyl-4-phenyl-5,6,7,8-tetrahydropyrazolo(3,4-b)(1,5)diazepin-1H,4H-5,7-dion

2,3 g 1-Isopropyl-3,8-dimethyl-5,6,7,8-tetrahydropyrazolo(3,4-b)(1,5)diazepin-1H,4H-5,7-dion (0,01 Mol), 1,5 g Kaliumacetat (0,015 Mol), 2,6 g Brombenzol (0,016 Mol), 1,8 g Kupferpulver und 130 ml Dimethylformamid werden 15 Stunden unter Rühren auf 150°C erhitzt. Die Mischung wird anschließend heiß abgesaugt, und das Lösungsmittel wird im Vakuum abgezogen. Der Rückstand wird in $CHCl_3$ aufgenommen, mit halbkonzentrierter Ammoniaklösung und anschließend mit Wasser gewaschen, getrocknet und eingedampft. Umkristallisation erfolgt aus Diisopropylether.
Fp.: 165°C.

## Beispiel 4

### 1-Ethyl-3-methyl-4-phenyl-5,6,7,8-tetrahydropyrazolo(3,4-b)(1,5)diazepin-1H,4H-5,7-dion

1-Ethyl-8-benzyl-3-methyl-4-phenyl-5,6,7,8-tetrahydropyrazolo(3,4-b)(1,5)diazepin-1H,4H-5,7-dion (500 mg) wird in 100 ml Ethanol gelöst und bei 100°C und 150 Atmosphären Wasserstoffdruck mit Platinoxid als Katalysator 100 Stunden hydriert. Danach wird der Katalysator abfiltriert, das Lösungsmittel im Vakuum eingedampft und der Rückstand mit $CHCl_3$/Ethanol (9 : 1) als Laufmittel über eine kurze Kieselgelsäule filtriert. Das Endprodukt fällt danach analysenrein an.
Fp.: 172°C.

Nach den beschriebenen Vorschriften wurden außerdem folgende Verbindungen hergestellt:

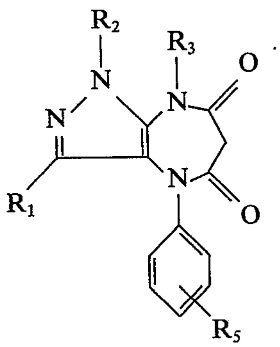

| Beispiel | $R_1$ | $R_2$ | $R_3$ | $R_5$ | Fp (°C) |
|---|---|---|---|---|---|
| 5 | $—CH_3$ | $—CH_3$ | $—CH_3$ | 3-Cl | 230 |
| 6 | $—CH_3$ | $—CH_3$ | $—CH_3$ | 2-Cl | 181 |
| 7 | $—CH_3$ | $—CH_3$ | $—CH_3$ | 4-$CH_3$ | 220 |
| 8 | $—CH_3$ | $—CH_3$ | $—CH_3$ | 4-F | 219 |
| 9 | $—CH_3$ | $—CH_3$ | $—CH_3$ | 4-Cl | 246 |
| 10 | $—CH_3$ | $—CH_3$ | $—CH_3$ | 3-F | 207 – 208 |
| 11 | $—CH_3$ | $—C_2H_5$ | $—CH_3$ | 2-Cl | 163 |
| 12 | $—CH_3$ | $—C_2H_5$ | $—CH_3$ | 4-F | 155 |
| 13 | $—CH_3$ | $—C_2H_5$ | $—CH_3$ | 4-Cl | 153 |
| 14 | $—CH_3$ | $—C_2H_5$ | $—CH_3$ | 4-$CH_3$ | 156 |
| 15 | $—CH_3$ | $—C_2H_5$ | $—CH_3$ | 3-Cl | 194 |
| 16 | $—CH_3$ | $—C_2H_5$ | $—CH_3$ | 3-$CH_3$ | 194 |
| 17 | $—CH_3$ | $—C_2H_5$ | $—CH_3$ | 3-F | 167 |
| 18 | $—CH_3$ | i-$C_3H_7$ | $—CH_3$ | 2-Cl | 229 |
| 19 | $—CH_3$ | i-$C_3H_7$ | $—CH_3$ | 2-$CH_3$ | 154 |
| 20 | $—CH_3$ | i-$C_3H_7$ | $—CH_3$ | 4-F | 198 |
| 21 | $—CH_3$ | i-$C_3H_7$ | $—CH_3$ | 3-Cl | 160 |
| 22 | $—CH_3$ | i-$C_3H_7$ | $—CH_3$ | 4-Cl | 196 |
| 23 | $—CH_3$ | i-$C_3H_7$ | $—CH_3$ | 2-F | 182 |
| 24 | $—CH_3$ | i-$C_3H_7$ | $—CH_2—CH_2—OCH_3$ | H | 136 |
| 25 | $—CH_3$ | i-$C_3H_7$ | $—CH_3$ | 3-F | 155 |
| 26 | $—CH_3$ | $CH_3$ | $—CH_3$ | 2-F | 181 |
| 27 | $—CH_3$ | $C_2H_5$ | $—CH_3$ | 2-F | 117 |
| 28 | $C_2H_5$ | $CH_3$ | $—CH_3$ | H | 181 |
| 29 | 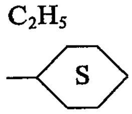 | $CH_3$ | $—CH_3$ | H | 223 |
| 30 | 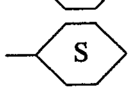 | $C_2H_5$ | $—CH_3$ | H | 163 |
| 31 | $CH_3$ | H | $—CH_3$ | H | 262 |
| 32 | n-$C_3H_7$ | $C_2H_5$ | $—CH_3$ | H | 129 |
| 33 | n-$C_3H_7$ | $CH_3$ | $—CH_3$ | H | 142 |

Fortsetzung

| Beispiel | $R_1$ | $R_2$ | $R_3$ | $R_5$ | Fp (°C) |
|---|---|---|---|---|---|
| 34 | $CH_3$ | 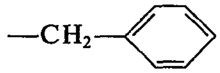 | $CH_3$ | H | 158 |
| 35 | $CH_3$ | $CH_3$ | 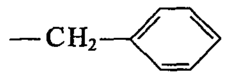 | H | 134 |
| 36 | H | $CH_3$ | $CH_3$ | H | 145 |
| 37 | $CH_3$ | $CH_3$ | H | H | 211 |
| 38 | $CH_3$ | $CH_3$ | $C_2H_5$ | H | 191 |
| 39 | $CH_3$ | $CH_3$ | n-$C_3H_7$ | H | 188 |
| 40 | $CH_3$ | $CH_3$ | 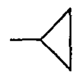 | H | 172 |
| 41 | $CH_3$ | $CH_3$ | 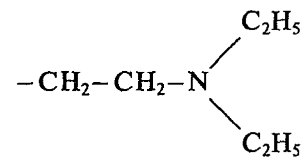 | H | 121 |

**Patentansprüche für die Vertragsstaaten: BE, CH, LI, DE, FR, GB, IT, NL, SE**

1. Verbindungen der allgemeinen Formel I

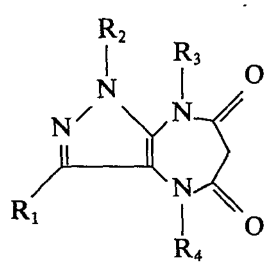

(I)

worin

$R_1$ und $R_2$ gleich oder verschieden sind und Wasserstoffatome, Alkylgruppen mit 1–6 C-Atomen darstellen, wobei einer der Reste $R_1$ und $R_2$ auch eine Benzyl-, Trifluormethyl- oder Phenylgruppe sein kann,

$R_3$ ein Wasserstoffatom, eine Alkylgruppe mit 1–6 C-Atomen, die gegebenenfalls durch eine Phenylgruppe, eine Alkoxygruppe mit 1–6 C-Atomen, eine Trifluormethyl- oder eine Dialkylaminogruppe mit 2–12 C-Atomen oder eine Cycloalkylgruppe mit 3–6 C-Atomen substituiert ist, eine Alkenyl- oder Alkinylgruppe mit 2–6 C-Atomen, eine Cycloalkylgruppe mit 3–6 C-Atomen, eine Carbalkoxygruppe mit 2–6 C-Atomen bedeutet und

$R_4$ eine Phenylgruppe oder eine ein- oder zweifach durch Methyl, Cl, Br, F, Nitro, Cyan und/oder Trifluormethyl substituierte Phenylgruppe

bedeutet.

2. Verbindungen der allgemeinen Formel I gemäß Anspruch 1, worin

$R_1$ $CH_3$,
$R_2$ Wasserstoff, $CH_3$, $C_2H_5$, i-$C_3H_7$ oder Phenyl,
$R_3$ $CH_3$,
$R_4$ Phenyl, ein- oder zweifach durch Chlor, Brom oder Fluor substituiertes Phenyl

bedeuten.

3. Verfahren zur Herstellung von Verbindungen gemäß Anspruch 1, dadurch gekennzeichnet, daß man ein 5,6,7,8-Tetrahydropyrazolo(3,4-b)(1,5)diazepin-1H,4H-5,7-dion der allgemeinen Formel II

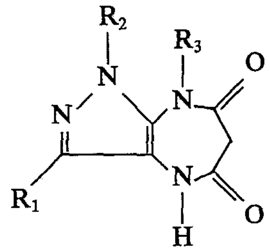

(II)

worin $R_1$, $R_2$ und $R_3$ (mit Ausnahme von Wasserstoff) die oben angegebenen Bedeutungen besitzen, mit einer Verbindung der allgemeinen Formel III

$R_4X$ (III)

worin $R_4$ die oben angegebenen Bedeutungen hat und X ein Halogenatom ist, umsetzt.

4. Verfahren zur Herstellung von Verbindungen gemäß Anspruch 1, in denen $R_3$ Wasserstoff bedeutet, dadurch gekennzeichnet, daß man eine Verbindung der allgemeinen Formel I, in der $R_3$ Alkoxymethyl oder Aralkoxymethyl bedeutet, der Hydrolyse oder eine Verbindung der allgemeinen Formel I, in der $R_3$ Benzyl bedeutet, der Hydrogenolyse unterwirft.

5. Verfahren zur Herstellung pharmazeutischer Zubereitungen mit anxiolytischer und antikonvulsiver Wirkung, dadurch gekennzeichnet, daß man Verbindungen gemäß Anspruch 1 gegebenenfalls zusammen mit pharmazeutischen Trägern oder Hilfsstoffen in eine für therapeutische Zwecke geeignete Anwendungsform bringt.

6. Pharmazeutische Präparate mit anxiolytischer und antikonvulsiver Wirkung, enthaltend als Wirkstoff eine oder mehrere Verbindungen der allgemeinen Formel I in Mengen von 1—50 mg, vorzugsweise 5—25 mg/Doses im Gemisch mit üblichen pharmazeutischen Hilfs- und Trägerstoffen.

**Patentansprüche für Österreich**

1. Verfahren zur Herstellung von Verbindungen der allgemeinen Formel I

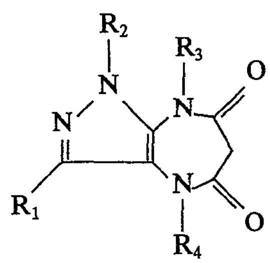

(I)

worin

| | |
|---|---|
| $R_1$ und $R_2$ | gleich oder verschieden sind und Wasserstoffatome, Alkylgruppen mit 1—6 C-Atomen darstellen, wobei einer der Reste $R_1$ und $R_2$ auch eine Benzyl-, Trifluormethyl- oder Phenylgruppe sein kann, |
| $R_3$ | ein Wasserstoffatom, eine Alkylgruppe mit 1—6 C-Atomen, die gegebenenfalls durch eine Phenylgruppe, eine Alkoxygruppe mit 1—6 C-Atomen, eine Trifluormethyl- oder eine Dialkylaminogruppe mit 2—12 C-Atomen oder eine Cycloalkylgruppe mit 3—6 C-Atomen substituiert ist, eine Alkenyl- oder Alkinylgruppe mit 2—6 C-Atomen, eine Cycloalkylgruppe mit 3—6 C-Atomen, eine Carbalkoxygruppe mit 2—6 C-Atomen bedeutet und |
| $R_4$ | eine Phenylgruppe oder eine ein- oder zweifach durch Methyl, Cl, Br, F, Nitro, Cyan und/oder Trifluormethyl substituierte Phenylgruppe |

bedeutet, dadurch gekennzeichnet, daß man ein 5,6,7,8-Tetrahydropyrazolo(3,4-b)(1,5)diazepin-1H,4H-5,7-dion der allgemeinen Formel II

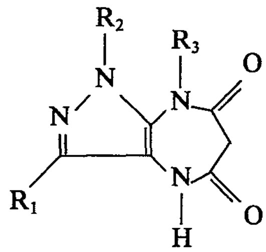

(II)

worin $R_1$, $R_2$ und $R_3$ (mit Ausnahme von Wasserstoff) die oben angegebenen Bedeutungen besitzen, mit einer Verbindung der allgemeinen Formel III

$R_4X$ (III)

worin $R_4$ die oben angegebenen Bedeutungen hat und X ein Halogenatom ist, umsetzt.

2. Verfahren zur Herstellung von Verbindungen gemäß Anspruch 1, in denen $R_3$ Wasserstoff bedeutet, dadurch gekennzeichnet, daß man eine Verbindung der allgeminen Formel I, in der $R_3$ Alkoxymethyl oder Aralkoxymethyl bedeutet, der Hydrolyse oder eine Verbindung der allgemeinen Formel I, in der $R_3$ Benzyl bedeutet, der Hydrogenolyse unterwirft.

3. Verfahren zur Herstellung pharmazeutischer Zubereitungen mit anxiolytischer und antikonvolsiver Wirkung, dadurch gekennzeichnet, daß man Verbindungen gemäß Anspruch 1 gegebenenfalls zusammen mit pharmazeutischen Trägern oder Hilfsstoffen in eine für therapeutische Zwecke geeignete Anwendungsform bringt.

**Claims for the contracting States: BE, CH, LI, DE, FR, GB, IT, NL, SE**

1. Compounds of the general formula I

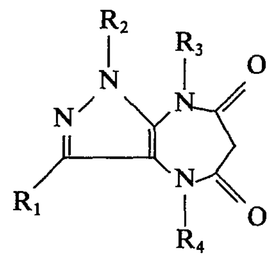

(I)

in which

| | |
|---|---|
| $R_1$ and $R_2$ | are identical or different and represent hydrogen atoms or alkyl groups with 1–6 C atoms, it also being possible for one of the radicals $R_1$ and $R_2$ to be also a benzyl, trifluoromethyl or phenyl group, |
| $R_3$ | denotes a hydrogen atom, an alkyl group which has 1–6 C atoms and is optionally substituted by phenyl group, an alkoxy group with 1–6 C atoms, a trifluoromethyl group, a dialkylamino group with 2–12 C atoms or a cycloalkyl group with 3–6 C atoms, an alkenyl or alkinyl group with 2–6 C atoms, a cycloalkyl group with 3–6 C atoms or a carbalkoxy group with 2–6 C atoms, |
| $R_4$ | is a phenyl group or a phenyl group which is monosubstituted or disubstituted by methyl, Cl, Br, F, nitro, cyano and/or trifluoromethyl. |

2. Compounds according to claim 1, wherein

$R_1$ is $CH_3$,
$R_2$ is hydrogen, $CH_3$, $C_2H_5$, i-$C_3H_7$ or phenyl,
$R_3$ is $CH_3$,
$R_4$ is phenyl or phenyl monosubstituted or disubstituted by chlorine, bromine or fluorine.

3. Process for the preparation of compounds according to claim 1, characterized by reacting a 5,6,7,8-tetrahydropyrazolo(3,4-b)(1,5)-diazepine-1H,4H-5,7-dione of the general formula II

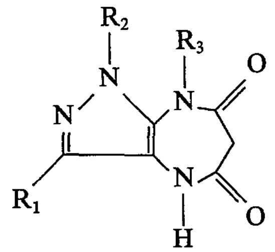

(II)

in which $R_1$, $R_2$, $R_3$ (with the exception of hydrogen) have the above mentioned meaning, with a compound of the general formula III

$R_4X$ (III)

in which $R_4$ has the above mentioned meaning and X is a halogen atom.

4. Process for the preparation of compounds according to claim 1, in which $R_3$ denotes hydrogen, characterized by subjecting a compound of the general formula I in which $R_3$ denotes alkoxymethyl or aralkoxymethyl to hydrolysis or subjecting a compound of the general formula I in which $R_3$ denotes benzyl to hydrogenolysis.

5. Process for the production of pharmaceutical formulations with an anxiolytic and anticonvulsive action, characterized by bringing compounds according to claim 1 into a use form suitable for therapeutic purposes, if appropriate together with pharmaceutical excipients or auxiliaries.

6. Pharmaceutical preparations with anxiolytic and anticonvulsive action containing as active ingredient at least one compound of the general formula I in an amount of from 1 to 50 mg, preferably 5 to 25 mg/dose in admixture with the usual pharmaceutical excipients and auxiliaries.

**Claims for contracting State: Austria**

1. Process for the preparation of compounds of the general formula I

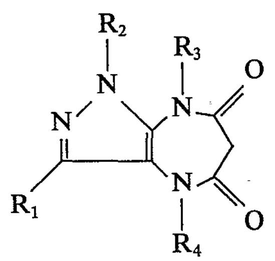

(I)

in which

| | |
|---|---|
| $R_1$ and $R_2$ | are identical or different and represent hydrogen atoms or alkyl groups with 1–6 C atoms, it also being possible for one of the radicals $R_1$ and $R_2$ to be also a benzyl, trifluoromethyl or phenyl group, |
| $R_3$ | denotes a hydrogen atom, an alkyl group which has 1–6 C atoms and is optionally substituted by a phenyl group, an alkoxy group with 1–6 C atoms, a trifluoromethyl group, a dialkylamino group with 2–12 C atoms or a cycloalkyl group with 3–6 C atoms, an alkenyl or alkinyl group with 2–6 C atoms, a cycloalkyl group with 3–6 C atoms or a carbalkoxy group with 2–6 C atoms, |
| $R_4$ | is a phenyl group or a phenyl group which is monosubstituted or disubstituted by methyl, Cl, Br, F, nitro, cyano and/or trifluoromethyl, |

characterized by reacting a 5,6,7,8-tetrahydropyrazolo(3,4-b)(1,5)-diazepine-1H,4H-5,7-dione of the general formula II

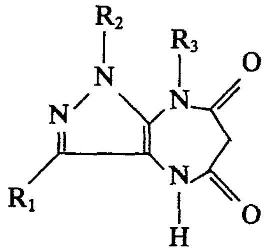

(II)

in which $R_1$, $R_2$, $R_3$ (with the exception of hydrogen) have the above mentioned meaning, with a compound of the general formula III

$R_4X$ (III)

in which $R_4$ has the above mentioned meaning and X is a halogen atom.

2. Process for the preparation of compounds according to claim 1 in which $R_3$ denotes hydrogen, characterized by subjecting a compound of the genereal formula I in which $R_3$ denotes alkoxymethyl or aralkoxymethyl to hydrolysis or subjecting a compound of the general formula I in which $R_3$ denotes benzyl to hydrogenolysis.

3. Process for the production of pharmaceutical formulations with an anxiolytic and anticonvulsive action, characterized by bringing compounds according to claim 1 into a use form suitable for therapeutic purposes, if appropriate together with pharmaceutical excipients or auxiliaries.

**Revendications pour l'Etats Contractants: BE, CH, LI, DE, FR, GB, IT, NL, SE**

1. Composés répondant à la formule générale I

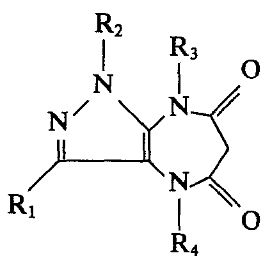

(I)

dans laquelle:

$R_1$ et $R_2$ sont identiques ou différents et représentent l'hydrogène, des groupes alcoyle en $C_1$ à $C_6$, un des radicaux $R_1$ et $R_2$ pouvant aussi être un groupe benzyle, trifluorométhyle ou phényle,

$R_3$ est un atome d'hydrogène, un groupe alcoyle en $C_1$ à $C_6$, qui est éventuellement substitué par un groupe phényle, un groupe alcoxy en $C_1$ à $C_6$, un groupe trifluorométhyle ou un groupe dialcoylamino en $C_2$ à $C_{12}$ ou un groupe cycloalcoyle en $C_3$ à $C_6$; un groupe alcényle ou alcinyle en $C_2$ à $C_6$, un groupe cycloalcoyle en $C_3$ à $C_6$, un groupe carbalcoxy en $C_2$ à $C_6$, et

$R_4$ désigne un groupe phényle ou un groupe phényle substitué une ou deux fois par un groupe méthyle, Cl, Br, F, nitro, cyano et/ou trifluorométhyle.

2. Composés répondant à la formule générale I suivant la revendication 1, dans laquelle

$R_1$ désigne $CH_3$,
$R_2$ l'hydrogène, $CH_3$, $C_2H_5$, i-$C_3H_7$ ou un phényle,
$R_3$ $CH_3$,
$R_4$ un phényle, un phényle substitué une ou deux fois par le chlore, le brome ou le fluor.

3. Procédé de préparation de composés suivant la revendication 1, caractérisé en ce qu'on fait réagir une 5,6,7,8-tétrahydropyrazolo(3,4-b)(1,5)diazépin-1H,4H-5,7-dione répondant à la formule générale II:

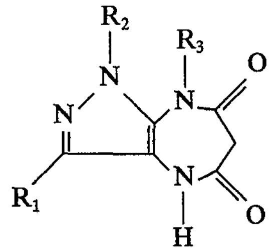

(II)

dans laquelle $R_1$, $R_2$ et $R_3$ (à l'exception de l'hydrogène) possèdent les significations indiquées ci-dessus, avec un composé répondant à la formule générale III:

$R_4X$ (III)

dans laquelle $R_4$ a les significations indiquées ci-dessus et X est un atome d'halogène.

4. Procédé de préparation de composés suivant la revendication 1, dans lesquels $R_3$ désigne l'hydrogène, caractérisé en ce qu'on soumet un composé répondant à la formule générale I, dans laquelle $R_3$ est un alcoxyméthyle ou un aralcoxyméthyle, à une hydrolyse, ou un composé répondant à la formule générale I, dans laquelle $R_3$ désigne un benzyle, à une hydrogénolyse.

5. Procédé de préparation de préparations pharmaceutiques ayant une action anxiolytique et anticonvulsivante, caractérisé en ce qu'on met des composés suivant la revendication 1, éventuellement avec des supports ou adjuvants pharmaceutiques, sous une forme d'utilisation appropriée à des fins thérapeutiques.

6. Préparations pharmaceutiques ayant une action anxiolytique et anticonvulsivante, contenant comme substance active un ou plusieurs composés répondant à la formule générale I, à raison de 1 à 50 mg, de préférence à raison de 5 à 25 mg/dose, en mélange avec des adjuvants et supports pharmaceutiques habituels.

**Revendications pour l'Etat Contractant: Autriche**

1. Procédé de préparation de composés répondant à la formule générale I:

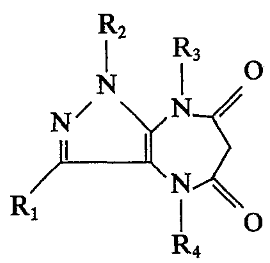

(I)

dans laquelle:

$R_1$ et $R_2$ sont identiques ou différents et représentent l'hydrogène, des groupes alcoyle en $C_1$ à $C_6$, un des radicaux $R_1$ et $R_2$ pouvant aussi être un groupe benzyle, trifluorométhyle ou phényle,

$R_3$ est un atome d'hydrogène, un groupe alcoyle en $C_1$ à $C_6$, qui est éventuellement substitué par un groupe phényle, un groupe alcoxy en $C_1$ à $C_6$, un groupe trifluorométhyle ou un groupe dialcoylamino en $C_2$ à $C_{12}$ ou un groupe cycloalcoyle en $C_3$ à $C_6$; un groupe alcényle ou alcinyle en $C_2$ à $C_6$, un groupe cycloalcoyle en $C_3$ à $C_6$, un groupe carbalcoxy en $C_2$ à $C_6$, et

$R_4$ désigne un groupe phényle ou un groupe phényle substitué une ou deux fois par un groupe méthyle, Cl, Br, F, nitro, cyano et/ou trifluorométhyle,

caractérisé en ce qu'on fait réagir une 5,6,7,8-tétrahydropyrazolo(3,4-b)(1,5)diazépin-1H,4H-5,7-dione répondant à la formule générale II:

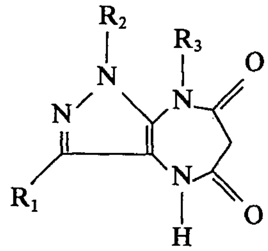

(II)

dans laquelle $R_1$, $R_2$ et $R_3$ (à l'exception de l'hydrogène) possèdent les significations indiquées ci-dessus, avec un composé répondant à la formule générale III:

$$R_4X \qquad \text{(III)}$$

dans laquelle $R_4$ a les significations indiquées ci-dessus et X est un atome d'halogène.

2. Procédé de préparation de composés suivant la revendication 1, dans lesquels $R_3$ désigne l'hydrogène, caractérisé en ce qu'on soumet un composé répondant à la formule générale I, dans laquelle $R_3$ est un alcoxyméthyle ou un aralcoxyméthyle, à une hydrolyse, ou un composé répondant à la formule générale I, dans laquelle $R_3$ désigne un benzyle, à une hydrogénolyse.

3. Procédé de préparation de préparations pharmaceutiques ayant une action anxiolytique et anticonvulsivante, caractérisé en ce qu'on met des composés suivant la revendication 1, éventuellement avec des supports ou adjuvants pharmaceutiques, sous une forme d'utilisation appropriée à des fins thérapeutiques.